(19) Europäisches Patentamt
European Patent Office
Office européen des brevets

(11) **EP 4 799 686 A1**

(12) **EUROPEAN PATENT APPLICATION**

(43) Date of publication:
**02.09.2026 Bulletin 2026/36**

(21) Application number: **25210291.8**

(22) Date of filing: **22.10.2025**

(51) International Patent Classification (IPC):
***A61P 3/10*** $^{(2006.01)}$ ***A61K 9/28*** $^{(2006.01)}$
***A61K 31/4985*** $^{(2006.01)}$ ***A61K 31/155*** $^{(2006.01)}$

(52) Cooperative Patent Classification (CPC):
**A61P 3/10; A61K 9/282; A61K 31/4985;**
A61K 31/155

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **28.02.2025 TR 2025002461**

(71) Applicant: **Sanovel Ilac Sanayi Ve Ticaret Anonim Sirketi**
**Istanbul (TR)**

(72) Inventors:
- **SUNEL, Fatih**
  **Istanbul (TR)**
- **ATAK, Fadime Bilgehan**
  **Istanbul (TR)**
- **PEHLIVAN AKALIN, Nur**
  **Istanbul (TR)**
- **TUNC, Guldeniz**
  **Istanbul (TR)**

(74) Representative: **Genç Ilhan, Oznur**
**Istanbul Patent A.S.**
**Plaza 33, Buyukdere Cad. No: 33/16**
**Sisli**
**34381 Istanbul (TR)**

(54) **A MULTILAYER TABLET COMPRISING METFORMIN AND SITAGLIPTIN**

(57) The present invention relates to a film coated tablet formulation comprising a core tablet having metformin hydrochloride and at least one matrix agent and intermediate layer surrounding the core tablet and a coating solution having sitagliptin surrounding the core tablet wherein the intermediate layer is between 1.3% and 3.1% by weight in the total tablet. Further, the present invention provides a method for the preparation of said composition.



Processed by Luminess, 75001 PARIS (FR)

## Description

### Field of the invention

The present invention relates to a film coated tablet formulation comprising a core tablet having metformin hydrochloride and at least one matrix agent and intermediate layer surrounding the core tablet and a coating solution having sitagliptin surrounding the core tablet wherein the intermediate layer is between 1.3% and 3.1% by weight in the total tablet. Further, the present invention provides a method for the preparation of said composition.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy.

In Type 2 diabetes, the pancreas makes insulin, but it either doesn't produce enough, or the insulin does not work properly. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether, and chloroform. The chemical name of metformin is 1,1-dimethyl biguanide, has the following chemical structure of Formula I.

NH NH

$H_3C$–N(–$CH_3$)–C(=NH)–NH–C(=NH)–$NH_2$

Formula I

Although metformin is effective at lowering blood glucose levels, its use is associated with gastrointestinal (GI) adverse effects, particularly diarrhea and nausea. These adverse effects may limit the tolerated dose of metformin and cause patients to discontinue the therapy.

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

The chemical name of sitagliptin is (3R)-3-amino-1-[3-(trifluoromethyl)-6,8-dihydro-5H-[1,2,4]triazolo[4,3-a] pyrazin-7-yl]-4-(2,4,5-trifluorophenyl) butan-1-one or (2R)-4-oxo-4- [3-(trifluoromethyl)-5,6-dihydro[1,2,4]triazolo[4,3-a] pyrazin-7(8H)-yl]-1-(2,4,5trifluorophenyl) butan-2-amine) and its chemical structure is shown in the Formula II.

Formula II

Sitagliptin is disclosed in the U.S. Patent No. 6699871. A crystal phosphate monohydrate form of sitagliptin is disclosed in the patent WO 2005003135.

DPP-4 inhibitors, especially sitagliptin have a novel mechanism of action and many studies showing their efficacy and safety in medication are available.

Sitagliptin increases plasma GLP-1 concentration and elevates cellular cAMP levels in pancreatic beta-cells leading to potentiate insulin secretion, whereas metformin improves glucose tolerance in patients with Type 2 diabetes, lowering both basal and postprandial plasma glucose. Its pharmacologic mechanisms of action are different from other classes of oral antihyperglycemic agents in that metformin decreases hepatic glucose production, decreases intestinal absorption of glucose and improves insulin sensitivity by increasing peripheral glucose uptake and utilization. Therefore, they both help to reduce blood glucose levels in different pathways in type 2 diabetes patients.

Extended-release formulations of metformin have advantages over immediate release in terms of affording a more uniform maintenance of blood plasma active drug concentrations and providing better patient compliance by reducing the frequency of administration required.

A tablet formulation for sitagliptin and extended-release metformin combination is commercially available under the trade name Janumet XR®.

PCT publication WO 2009111200 discloses the tablet comprising the combinations of an extended-release form of metformin, or a pharmaceutically acceptable salt thereof, coated with an immediate-release form of sitagliptin, or a pharmaceutically acceptable salt thereof.

WO 2009099734 discloses pharmaceutical compositions providing an extended release of metformin and an immediate release of sitagliptin. The tablet core is comprised of metformin and an extended release polymer (HPMC). The tablet core is then coated with immediate release polymer comprising sitagliptin.

There is also still a need for a film coated tablet form comprising an extended-release formulation for metformin and immediate release formulation for sitagliptin having desired dissolution profile and stability.

In the present invention, the dosage form of pharmaceutical combination is a core tablet coating comprising metformin with an intermediate layer and a solution coating comprising sitagliptin to overcome the problem of incompatibility and dissolution rate with a formulation appropriate to the characteristics of the active ingredients.

## Detailed description of the Invention

The main object of the present invention is to avoid incompatibility problems between metformin and sitagliptin, and adding additional advantages to the prior art.

It is a further object of the present invention to provide a film coated tablet formulation comprising delivery of a first active drug as an extended-release formulation for metformin in combination with delivery of a second active drug by immediate release comprising sitagliptin which having the desired dissolution profile and stability.

Another object of the present invention is to provide a process for preparing a tablet comprising metformin and sitagliptin. The process is a simple, rapid, cost effective, timesaving, and industrially convenient method.

As used herein, the term "sitagliptin" includes sitagliptin and pharmaceutically acceptable salts thereof. The pharmaceutically acceptable salts comprise hydrochloride, hydrobromide, phosphate, sulphate, mesylate, besylate, tosylate, fumarate, malonate, malate, succinate, lactate, glycolate, maleate, citrate, and aspartate. The pharmaceutically acceptable salts of sitagliptin may be present in hydrous or anhydrous forms. The hydrate forms may be monohydrate or dihydrate forms. The pharmaceutically acceptable salts of sitagliptin may also be present in crystalline or amorphous forms. In this invention, preferably sitagliptin phosphate monohydrate is used.

The term "core" as used herein refers to a compact mass having a definite geometric shape such as tablets, granules, pellets, capsules.

The invention; In order to prevent possible compatibility problems that may occur while the active ingredients of Sitagliptin and Metformin HCl in the pharmaceutical form are presented in a single dosage form, it contains an intermediate

layer that separates the active ingredients from each other. The intermediate layer used within the scope of the invention also made the core tablet physically durable and prevented friability problems that may occur during the coating of the Sitagliptin active ingredient. For this purpose, we compressed a mixture comprising metformin as a core tablet, then we coated it with an intermediate layer, then we coated it with after a solution comprising sitagliptin. Thanks to the intermediate layer and its weight ratio on the tablet, sitagliptin and metformin are stable. The combination does not show incompatibilities, degradation problems, or extraction problems.

In the invention, when sitagliptin or a pharmaceutically acceptable salts thereof and at least one coating agent and at least one antioxidant is dissolved in a coating solvent, a solution comprising a higher proportion and homogeneity of sitagliptin or a pharmaceutically acceptable salts thereof is obtained, this minimizes active substance losses during process and provides more homogeneous (content uniformity) and efficient tablet formulation.

Metformin HCl is used big proportion that can lead to considerable problems during the preparation of formulation with regard to the uniformity of the content of active agent in the individual composition units. Because of problems uniformity of the content, the active substance may interact with several excipients. It reflects that content uniformity play important role in the dissolution of the drug. Also, in terms of physical properties, the compressibility of metformin is poor, and how to obtain a tablet or a capsule having an acceptable mechanical strength is an important problem in formulation development. Therefore, a mixture containing metformin was used. This mixture represents a mixture that has remained in the form of a granule with a direct mixture.

Furthermore, in order to shorten the process time within the scope of the invention, the active ingredient Metformin HCl was used in its mixture form. In this way, the extended release Metformin HCl core tablet is formed by the direct mixing method.

According to one embodiment of the present invention, a film coated tablet formulation comprises;

- A core tablet comprising a mixture comprising metformin hydrochloride and at least one matrix agent,
- Intermediate layer surrounding the core tablet,
- A coating solution comprising sitagliptin phosphate monohydrate surrounding the core tablet,
- A film coating

wherein intermediate layer is between 1.3% and 3.1% by weight in the total tablet.

According to one embodiment of the present invention, the weight of intermediate layer is between 1.7% and 2.7% by weight in the total tablet.

According to one embodiment of the present invention, the amount of the mixture comprising metformin hydrochloride is between 40.0% and 70.0% by weight in the total tablet.

According to one embodiment of the present invention, the amount of metformin hydrochloride is between 90.0% and 97.0% by weight in the mixture. Preferably, it is 95.0% by weight in the mixture (metformin granulate Direct Compressible grade %95).

As used here in, 'particle size' means the cumulative volume size distribution as tested by any conventionally accepted method such as the laser diffraction method (i.e. malvern analysis). The term d (0.5) means, the size at which 50% by volume of the particles are finer, The term d (0.6) means, the size at which 60% by volume of the particles are finer,

According to this embodiment of the present invention, sitagliptin has a d50 particle size between 27 and 70 μm, preferably between 35 μm and 50 μm.

According to this embodiment of the present invention, Metformin DC granule has a d60 particle size between 40 and 100 μm, preferably between 55 μm and 85 μm.

According to one embodiment of the present invention, the compartments are in direct contact with each other. In this invention, the compartments are in the form of core or coating. The first compartment is a tablet core which comprises metformin hydrochloride. The second compartment is intermediate layer, the third compartment is sitagliptin solution coating, the fourth compartment is the film coating.

According to one embodiment of the present invention, the amount of sitagliptin is between 3.0% and 15.0% by weight of the total tablet. Preferably, the amount of sitagliptin is between 3.5% and 10.0% by weight of the total tablet.

Said matrix agent is selected from the group comprising hydroxypropyl methyl cellulose, hydroxyethyl cellulose, hydroxypropyl cellulose, methyl cellulose, galactomannanes, guar gum, carob gum, alginates, polyvinylacetate, acrylic acid polymers, polyethylene oxide, white wax, bees wax, carnauba wax, stearic acid, palmitic acid, lauric acid, cetyl alcohol, cetostearyl alcohol, stearyl alcohol, ethyl cellulose, acrylic acid polymers and copolymers (available commercially under Eudragit<®>brand) or mixtures thereof.

According to one embodiment of the present invention, the matrix agent is hydroxypropyl methyl cellulose K100 MCR/ K100M/ CN10T. The flowability and compressibility of metformin is poor. In the present invention, granulating metformin with the help of selected matrix agent provides desired flowability and compressibility of metformin.

According to one embodiment of the present invention, the amount of matrix agent is between 15.0% and 30.0% by weight of the total tablet.

According to one embodiment of the present invention, a coating solution comprising sitagliptin phosphate monohydrate further comprises at least one antioxidant and at least one coating agent.

Suitable antioxidants are selected from the group comprising propyl gallate, alpha tocopherol, ascorbic acid, ascorbyl palmitate, butylhydroxyanisole (BHA), butylhydroxytoluene (BHT), erythorbic acid, monothioglycerol, potassium metabisulfite, sodium ascorbate, sodium metabisulfite, sodium sulfite, thymol or mixtures thereof.

According to one embodiment of the present invention, antioxidant is propyl gallate.

According to one embodiment of the present invention, the amount of antioxidant is between 0.01% and 2.0% by weight of the total tablet.

According to one embodiment of the present invention, the coating agents used in the coating solution comprising sitagliptin are polyethylene glycol, hydroxypropyl methyl cellulose, kaolin or mixture thereof.

According to one embodiment of the present invention, the core tablet further comprises at least one diluent.

Suitable diluent is selected from group comprising microcrystalline cellulose, lactose anhydrous, dicalcium phosphate dihydrate, ammonium alginate, calcium carbonate, calcium phosphate, calcium sulfate, cellulose, cellulose acetate, dextrates, dextrin, dextrose, erythritol, ethylcellulose, mannitol, magnesium carbonate, magnesium oxide, maltodextrin, polydextrose, polymethacrylates, sodium alginate, sodium chloride, starch, sugar spheres, sulfobutylether beta-cyclodextrin, polysorbate 80, xylitol or mixtures thereof.

According to one embodiment of the present invention, the amount of diluent is between 5.0% and 18.0%, preferably it is between 6.0% and 15.0% by weight of the total tablet.

According to one embodiment of the present invention, the diluent is microcrystalline cellulose.

According to one embodiment of the present invention, the core tablet further comprises at least one lubricant.

Suitable lubricants are selected from group comprising magnesium stearate, hydrophobic silicon dioxide, talc, sodium stearyl fumarate, polyethylene glycol, sodium lauryl sulphate, magnesium lauryl sulphate, fumaric acid, glyceryl palmitostearate, hydrogenated natural oils, zinc stearate, calcium stearate, silica, stearic acid, paraffin or mixtures thereof.

According to one embodiment of the present invention, the amount of lubricants is between 0.1% and 4.0% by weight of the total tablet.

According to one embodiment of the present invention, the lubricant is sodium stearyl fumarate.

According to one embodiment of the present invention, an intermediate layer comprises at least one coating agent.

Suitable coating agents are selected from the group comprising polymethacrylates, polydextrose, polyalkylacrylates copolymers, triacetin, hydroxylpropyl methyl cellulose, medium chain triglycerides, kaolin, hydroxypropyl cellulose, carnauba wax, polyvinyl alcohol, polyethylene glycol, talc, polyvinyl alcohol-polyethylene glycol copolymers (Kollicoat® IR), ethylcellulose dispersions (Surelease®), polyvinylprolidone, polyvinylprolidone-vinyl acetate copolymer (PVP-VA), all kinds of Opadry®, pigments, dyes, titanium dioxide, red iron oxide, black iron oxide or mixtures thereof.

According to one embodiment of the present invention, the film coating is prepared with coating agents. Used the coating agents in the film coating are polyethylene glycol, hydroxypropyl cellulose, titanium dioxide, indigo dye, carnauba wax, or mixture thereof.

According to one embodiment of the present invention, the ratio of the core tablet comprising a mixture comprising metformin hydrochloride to the coating solution comprising sitagliptin is in the range of between 4 and 11 by weight. This ratio provides desired dissolution profile of active agents and desired combination efficacy.

According to this embodiment of the invention, the core tablet is between 75.0% and 87.0%, preferably between 78.0% and 84.0% by weight of the total tablet.

According to this embodiment of the invention, the coating solution comprising sitagliptin is between 8.0% and 20.0%, preferably between 10.0% and 17.0% by weight of the total tablet.

According to one embodiment of the present invention, a film coated tablet formulation comprises;

- A core tablet comprising metformin HCl, hydroxypropyl methylcellulose K100 MCR/ K100M/ CN10T as matrix agent, sodium stearyl fumarate as lubricant,
- An intermediate layer comprising coating agents,
- A coating solution comprising sitagliptin phosphate monohydrate surrounding the core tablet having propyl gallate as antioxidant and coating agents,
- A film coating.

According to one embodiment of the present invention, a film coated tablet formulation comprises;

- A core tablet comprising metformin HCl, microcrystalline cellulose, hydroxypropyl methylcellulose K100 MCR/ K100M/ CN10T as matrix agent, sodium stearyl fumarate as lubricant,
- An intermediate layer comprising coating agents,
- A coating solution comprising sitagliptin phosphate monohydrate surrounding the core tablet having propyl gallate as

antioxidant and coating agents,
- A film coating.

According to another embodiment of the present invention, there is provided a process for preparation of the film coated tablet formulation comprising metformin and sitagliptin, wherein the tablet is prepared by a process comprising the steps of:

a) preparing a core comprising metformin HCl which is obtained wet granulation or direct compression,
b) applying an intermediate layer surrounding the core,
c) applying a coating solution comprising sitagliptin phosphate monohydrate surrounding the core which is obtained wet granulation,
d) applying a film coating.

Example 1;

| | | (%) amount (w/w) | (%) amount (w/w) |
|---|---|---|---|
| **Core** | Metformin HCl granule DC %95 | 40.0 - 70.0 | 47.8 |
| | Microcrystalline cellulose PH 102 | 5.0 – 18.0 | 9.33 |
| | Hydroxypropyl methylcellulose K100 MCR/ K100M/ CN10T | 15.0 – 30.0 | 25.54 |
| | Sodium stearyl fumarate | 0.1 – 4.0 | 0.54 |
| **Intermediate layer** | Opadry Clear<br>- Polyethylene glycol<br>- Kaolin<br>- Hydroxypropyl methyl cellulose | 0.5 – 4.0 | 2.27 |
| **Sitagliptin solution coating** | Sitagliptin phosphate monohydrate | 3.0 – 15.0 | 5.8 |
| | Propyl gallate | 0.01 - 2.0 | 0.11 |
| | Opadry Clear<br>- Polyethylene glycol<br>- Kaolin<br>- Hydroxypropyl methyl cellulose | 2.0 - 10.0 | 5.77 |
| **Film coating** | **Film coating**<br>Hydroxypropyl Cellulose<br>Macrogol 3350<br>Titanium Dioxide<br>Carnauba wax<br>Indigo Dye | 1.0 – 4.0 | 2.7 |
| | **Total Tablet** | **100** | **100** |

**Example** 1

A process for preparing the tablet in example 1 comprises the following steps:

**Core tablet**

a) Mixing Metformin HCI granule DC %95, microcrystalline cellulose and hydroxypropyl methylcellulose,
b) Then, adding sodium stearyl fumarate and mixing,
c) Compressing the mixture to form a core.

**Intermediate layer**

d) Dissolving Opadry clear in water,
e) Coating the core with the solution,

**Sitagliptin solution coating**

f) Preparing a coating solution with a coating material (preferably, Opadry Clear comprising polyethylene glycol and kaolin and hydroxypropyl methyl cellulose),
g) Adding propyl gallate into the coating solution and mixing,
h) Adding sitagliptin and then obtained the coating solution comprising sitagliptin,
i) Coating the core tablet with the solution coating comprising sitagliptin.

**Film coating**

j) preparing a film coating with hydroxypropyl cellulose, macrogol 3350, titanium dioxide, carnauba wax, indigo dye,
k) Coating at step (i) coated tablet with the film coating.

**Example 2**

| | | Amount (w/w) | (%) amount (w/w) | (%) amount (w/w) |
|---|---|---|---|---|
| Core | Metformin HCl granule DC %95 | 40.0 - 70.0 | 68.7 | 63.1 |
| | Hydroxypropyl methylcellulose K100 MCR/ K100M/ CN10T | 15.0 – 30.0 | 17.8 | 16.3 |
| | Sodium stearyl fumarate | 0.1 – 4.0 | 0.39 | 0.39 |
| Intermediate layer | Opadry Clear<br>- Polyethylene glycol<br>- Kaolin<br>-Hydroxypropyl methyl cellulose | 0.5 – 4.0 | 2.28 | 2.1 |
| Sitagliptin solution coating | Sitagliptin phosphate monohydrate | 3.0 – 15.0 | 4.1 | 7.71 |
| | Propyl gallate | 0.01 - 2.0 | 0.08 | 0.15 |
| | Opadry Clear<br>- Polyethylene glycol<br>- Kaolin<br>-Hydroxypropyl methyl cellulose | 2.0 - 10.0 | 4.1 | 7.9 |
| Film coating | **Film coating**<br>Hydroxypropyl Cellulose<br>Macrogol 3350<br>Titanium Dioxide<br>Carnauba wax<br>Indigo Dye | 1.0 – 4.0 | 2.7 | 2.4 |
| | **Total Tablet** | **100** | **100** | **100** |

**Example 2**

A process for preparing the tablet in example 2 comprises the following steps:

**Core tablet**

a) Mixing Metformin HCI granule DC %95 and hydroxypropyl methylcellulose,
b) Then, adding sodium stearyl fumarate and mixing,
c) Compressing the mixture to form a core.

**Intermediate layer**

d) Dissolving Opadry clear in water,
e) Coating the core with the solution,

**Sitagliptin solution coating**

f) Preparing a coating solution with a coating material (preferably, Opadry Clear comprising polyethylene glycol and kaolin and hydroxypropyl methyl cellulose),
g) Adding propyl gallate into the coating solution and mixing,
h) Adding sitagliptin and then obtained the coating solution comprising sitagliptin,

i) Coating the core tablet with the solution coating comprising sitagliptin.

**Film coating**

j) preparing a film coating with hydroxypropyl cellulose, macrogol 3350, titanium dioxide, carnauba wax, indigo dye,
k) Coating at step (i) coated tablet with the film coating.

**Example 3; A mixture comprising metformin HCl %95 DC**

| Ingredients | % by weight | % by weight |
|---|---|---|
| Metformin HCl | 90.0-97.0 | 95.0 |
| Pregelatinized starch | 0.2-3.0 | 1.0 |
| Polyvinylpyrrolidone K30 | 0.5-4.0 | 1.30 |
| Polyvinylpyrrolidone K90 | 0.01-1.5 | 2.0 |
| Crospovidone | 0.01-1.5 | 0.5 |
| Magnesium stearate | 0.01-1.5 | 0.2 |
| **TOTAL** | **100** | **100** |

## Claims

1. A film coated tablet formulation comprises;

   - A core tablet comprising a mixture comprising metformin hydrochloride and at least one matrix agent,
   - An intermediate layer surrounding the core tablet,
   - A coating solution comprising sitagliptin phosphate monohydrate surrounding the core tablet,
   - A film coating

   wherein intermediate layer is between 1.3% and 3.1% by weight in the total tablet.

2. The film coated tablet according to claim 1, wherein the intermediate layer is between 1.7% and 2.7% by weight in the total tablet.

3. The film coated tablet according to claim 1, wherein the amount of metformin hydrochloride is between 90.0% and 97.0% by weight in the mixture.

4. The film coated tablet according to claim 1, wherein the matrix agent is selected from the group comprising hydroxypropyl methyl cellulose, hydroxyethyl cellulose, hydroxypropyl cellulose, methyl cellulose, galactomannanes, guar gum, carob gum, alginates, polyvinylacetate, acrylic acid polymers, polyethylene oxide, white wax, bees wax, carnauba wax, stearic acid, palmitic acid, lauric acid, cetyl alcohol, cetostearyl alcohol, stearyl alcohol, ethyl cellulose, acrylic acid polymers and copolymers (available commercially under Eudragit<®>brand) or mixtures thereof.

5. The film coated tablet according to claim 1, wherein the matrix agent is hydroxypropyl methyl cellulose K100 MCR/ K100M/ CN10T.

6. The film coated tablet according to claim 1, wherein the amount of matrix agent is between 15.0% and 30.0% by weight of the total tablet.

7. The film coated tablet according to claim 1, wherein the coating solution comprising sitagliptin phosphate monohydrate further comprises at least one antioxidant and at least one coating agent.

**8.** The film coated tablet according to claim 7, wherein antioxidants are selected from the group comprising propyl gallate, alpha tocopherol, ascorbic acid, ascorbyl palmitate, butylhydroxyanisole (BHA), butylhydroxytoluene (BHT), erythorbic acid, monothioglycerol, potassium metabisulfite, sodium ascorbate, sodium metabisulfite, sodium sulfite, thymol or mixtures thereof.

**9.** The film coated tablet according to claim 8, wherein antioxidant is propyl gallate.

**10.** The film coated tablet according to claim 1, wherein the core tablet further comprises at least one diluent.

**11.** The film coated tablet according to claim 10, wherein the diluent is selected from group comprising microcrystalline cellulose, lactose anhydrous, dicalcium phosphate dihydrate, ammonium alginate, calcium carbonate, calcium phosphate, calcium sulfate, cellulose, cellulose acetate, dextrates, dextrin, dextrose, erythritol, ethylcellulose, mannitol, magnesium carbonate, magnesium oxide, maltodextrin, polydextrose, polymethacrylates, sodium alginate, sodium chloride, starch, sugar spheres, sulfobutylether beta-cyclodextrin, polysorbate 80, xylitol or mixtures thereof.

**12.** The film coated tablet according to claim 11, wherein the diluent is microcrystalline cellulose.

**13.** The film coated tablet according to claim 1, wherein the core tablet further comprises at least one lubricant.

**14.** The film coated tablet according to claim 1, wherein the intermediate layer comprises at least one coating agent.

**15.** The film coated tablet according to any preceding claims, wherein a formulation comprises;

- A core tablet comprising metformin HCl granule DC %95, hydroxypropyl methylcellulose K100 MCR/ K100M/ CN10T as matrix agent, sodium stearyl fumarate as lubricant,
- An intermediate layer comprising coating agents,
- A coating solution comprising sitagliptin phosphate monohydrate surrounding the core tablet having propyl gallate as antioxidant and coating agents,
- A film coating.

# EUROPEAN SEARCH REPORT

Application Number
EP 25 21 0291

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P<br>Y,P | EP 4 563 144 A1 (SANOVEL ILAC SANAYI VE TICARET AS [TR]) 4 June 2025 (2025-06-04)<br>* claims 1-8, [0026-0036], examples 1 and 2 [0056-0065 * | 1-15<br>1-15 | INV.<br>A61P3/10<br>A61K9/28<br>A61K31/4985<br>A61K31/155 |
| A,P | EP 4 563 143 A1 (SANOVEL ILAC SANAYI VE TICARET AS [TR]) 4 June 2025 (2025-06-04)<br>* examples 1-3, claims 1-5 * | 1-15 | |
| Y | WO 2009/099734 A1 (MERCK & CO INC [US]; POURKAVOOS NAZANEEN [US]) 13 August 2009 (2009-08-13)<br>* examples 1-2; tables 1-2 * | 1-15 | |
| | | | TECHNICAL FIELDS SEARCHED (IPC) |
| | | | A61P<br>A61K |

The present search report has been drawn up for all claims

1

| Place of search | Date of completion of the search | Examiner |
|---|---|---|
| Munich | 9 March 2026 | Erdmann, Mona |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 25 21 0291

This annex lists the patent family members relating to the patent documents cited in the above-mentioned European search report. The members are as contained in the European Patent Office EDP file on
The European Patent Office is in no way liable for these particulars which are merely given for the purpose of information.

09-03-2026

| Patent document cited in search report | Publication date | Patent family member(s) | Publication date |
|---|---|---|---|
| EP 4563144 A1 | 04-06-2025 | EP 4563144 A1 | 04-06-2025 |
| | | EP 4566593 A1 | 11-06-2025 |
| EP 4563143 A1 | 04-06-2025 | NONE | |
| WO 2009099734 A1 | 13-08-2009 | AU 2009210641 A1 | 13-08-2009 |
| | | CA 2713361 A1 | 13-08-2009 |
| | | CN 101932241 A | 29-12-2010 |
| | | EP 2249643 A1 | 17-11-2010 |
| | | JP 2011510986 A | 07-04-2011 |
| | | US 2010330177 A1 | 30-12-2010 |
| | | WO 2009099734 A1 | 13-08-2009 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6699871 B
- WO 2005003135 A
- WO 2009111200 A
- WO 2009099734 A